# EUROPEAN PATENT APPLICATION

(11) **EP 1 273 586 A1**
(43) Date of publication of application: **08.01.2003**
(21) Application number: 01955653.9
(22) Date of filing: 10.08.2001
(51) Int. Cl.: C07D 499/18, C07D 499/87, C07D 499/86

(54) **PENICILLIN CRYSTAL AND PROCESS FOR PRODUCING THE SAME**

(30) Priority: 11.08.2000 JP 2000244314
(71) Applicant: OTSUKA CHEMICAL COMPANY, LIMITED, Osaka-shi, Osaka 540-0021 (JP); TAIHO PHARMACEUTICAL CO., LTD., Tokyo 101-0054 (JP)
(72) Inventor: SHIMABAYASHI, Akihiro, OTSUKA CHEMICAL Co. Ltd, Tokushima-shi, Tokushima 771-0193 (JP); KAWAHARA, Ichiro, OTSUKA CHEMICAL Co. Ltd, Tokushima-shi, Tokushima 771-0193 (JP)
(74) Representative: Barz, Peter
(86) International application number: JP0106896
(87) International publication number: WO02016371

(57) **Abstract**

The crystal of the invention is the crystal of diphenylmethyl 2-methyl-2-triazolylmethylpenam-3-carboxylate 1,1-dioxide which is stable substantially without decomposition or degradation of properties even when left to stand at a temperature of 5 to 35° C for a period of 1 year. The crystal of the invention can be prepared by adding diphenylmethyl 2-methyl-2-triazolylmethylpenam-3-carboxylate 1,1-dioxide represented by the formula (1) 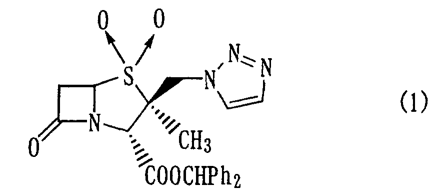 wherein Ph is a phenyl group, in the form of oily substance or in the form of amorphous powder or an organic solvent solution thereof to at least one species selected from alcohols and ketones to crystallize the diphenylmethyl 2-methyl-2-triazolylmethylpenam-3-carboxylate 1,1-dioxide.

## Description

### Field of the Invention

The present invention relates to crystalline penicillin and a process for preparing the same, and more specifically to diphenylmethyl 2-methyl-2-triazolylmethylpenam-3-carboxylate 1,1-dioxide crystal and a process for preparing the same.

### Background Art

Diphenylmethyl 2-methyl-2-triazolylmethylpenam-3-carboxylate 1,1-dioxide represented by the formula (1) (hereinafter referred to as "TAZB" where appropriate) 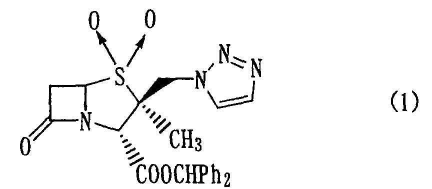 wherein Ph is a phenyl group is a compound useful as an intermediate for preparing tazobactam represented by the formula (2) 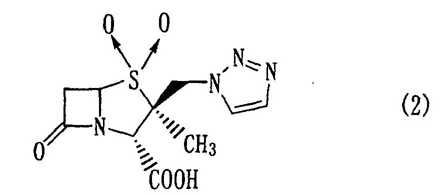 wherein Ph is as defined above.

Tazobactam has a very low antibacterial activity so that it is not used as an antibiotic per se. But it exhibits a beta-lactamases inhibitory activity when irreversibly bonded to beta-lactamase produced by microorganisms. For this reason, tazobactam may be used in mixture with known antibiotics prone to be inactivated by beta-lactamase to allow them to exhibit their inherent antibacterial activity against beta-lactamase-producing microorganisms (Katsuji SAKAI, Recent Antibiotics Manual, 10^{th} edition, page 113).

Tazobactam has a chemical structure of having 1,2,3-triazolylmethyl group in the 3-position. Tazobactam is prepared essentially via an intermediate for synthesis of tazobactam such as TAZB, p-nitrobenzyl 2-methyl-2-triazolylmethylpenam-3-carboxylate 1,1-dioxide or the like. Especially when using TAZB, a high-purity tazobactam can be prepared in a high yield by an industrially simple and inexpensive process.

Usually, TAZB is prepared in the form of oily substance, for example, by oxidizing diphenylmethyl 2-methyl-2-triazolylmethylpenam-3-carboxylate (hereinafter referred to as "TMPB") with an oxidizer in a solvent according to the process disclosed, e.g., in Japanese Examined Patent Publication No.121949/1995, especially in Example 5, and TAZB is produced in the form of amorphous powder by purification of oily substance with a silica gel column. Useful solvents include, for example, chloroform, pyridine, tetrahydrofuran, dioxane, acetone, methylene chloride, carbon tetrachloride, acetic acid, formic acid, dimethylformamide, water, mixtures thereof and the like. Useful oxidizers are, for example, permanganic acid, periodic acid, peracetic acid, trifluoroperacetic acid, perbenzoic acid, m-chloroperbenzoic acid, hydrogen peroxide and the like.

However, TAZB in the form of oily substance or in the form of amorphous powder obtained by the process disclosed in the foregoing publication is unstable since TMPB has a 1,2,3-triazole skeleton having a nucleophilic reactivity in the molecule. For example, when stored at ordinary temperature for a long time, the compound decomposes and markedly degrades in properties. It is desirable that an intermediate of pharmaceuticals maintains a high purity for a long time and can be stably handled without decomposition or degradation of properties under mild and economical conditions, e.g., storage at ordinary temperature. For this reason, TAZB in the form of oily substance or in the form of amorphous powder obtained by conventional processes is not favorable as an intermediate of pharmaceuticals.

Japanese Unexamined Patent Publication No.53462/1996, especially in Example 4, discloses that TAZB is produced in a yield of 95% by a process comprising reacting diphenylmethyl 2-methyl-2-aminomethylpenam-3-carboxylate 1,1-dioxide with 2,2-dichloroacetoaldehyde-p-toluenesulfonyl hydrazone in methanol at room temperature, concentrating the reaction mixture, dissolving the residue in methylene chloride, filtering the solution, concentrating the filtrate, and crystallizing the residue in a solvent mixture of ethyl acetate and n-hexane (1:1).

However, the TAZB prepared by such process does not exhibit a clear X-ray powder diffraction pattern, and is in the form of amorphous powder. This amorphous powder is unstable as described above and is likely to decompose and to degenerate when stored at room temperature (e.g. 5 to 35°C) for a long time.

### Disclosure of the Invention

An object of the present invention is to provide a TAZB substance which is highly stable and which is unlikely to decompose and to degrade in properties even when stored at room temperature for a long time.

Another object of the invention is to provide a process for preparing a TAZB substance which is highly stable and which is unlikely to decompose and to degrade in properties even when stored at room temperature for a long time.

The present inventors conducted extensive research to achieve these objects, and succeeded in producing TAZB crystal which is different in properties from known TAZB in the form of oily substance or in the form of amorphous powder. Based on this novel finding, the invention has been completed.

According to the invention, there is provided TAZB crystal (hereinafter referred to as "crystalline penicillin") characterized by having peaks at the following interplanar spacings in the X-ray powder diffraction pattern obtained by copper radiation of λ =1.5418 angstroms through a monochromator.

| d (interplanar spacing) |
|---|
| 10.412-11.508 |
| 6.864-7.586 |
| 5.193-5.740 |
| 4.911-5.428 |
| 4.668-5.159 |
| 4.443-4.911 |
| 4.152-4.590 |
| 4.081-4.510 |
| 3.738-4.131 |
| 3.549-3.922 |
| 3.072-3.395 |

According to the invention, there is provided a process for preparing crystalline penicillin, the process comprising the step of adding TAZB in the form of oily substance or in the form of amorphous powder or an organic solvent solution of such TAZB to at least one species selected from alcohols and ketones to crystallize the TAZB out of the solution.

The crystalline penicillin of the invention, although having a 1,2,3-triazole skeleton with a nucleophilic reactivity in the crystal molecule, is stable without decomposition or degradation of properties even when stored at room temperature for a period of one year or longer, and can retain the high purity, e.g. 95% or higher (especially 98% or higher). With this feature, the crystalline penicillin of the invention is very useful as an intermediate of tazobactam or like pharmaceuticals.

Using the crystalline penicillin of the invention, tazobactam having a purity of 99.9% or higher can be prepared in a yield of 95% or higher.

### TAZB of the Invention

The TAZB of the invention is represented by the formula (1) 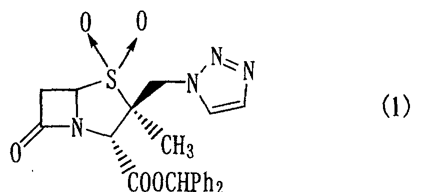 wherein Ph is as defined above.

The crystalline penicillin of the invention is composed of TAZB crystal having peaks in the above X-ray powder diffraction spectrum. An example includes the crystal having the X-ray powder diffraction spectrum shown below:

| d (interplanar spacing) | I/Io(relative intensity) |
|---|---|
| 10.412-11.508 | 0.42-0.45 |
| 6.864-7.586 | 1.00 |
| 5.193-5.740 | 0.39-0.87 |
| 4.911-5.428 | 0.47-0.92 |
| 4.668-5.159 | 0.14-0.20 |
| 4.443-4.911 | 0.15-0.18 |
| 4.152-4.590 | 0.25-0.34 |
| 4.081-4.510 | 0.19-0.43 |
| 3.738-4.131 | 0.21-0.33 |
| 3.549-3.922 | 0.25-0.34 |
| 3.072-3.395 | 0.22-0.31 |

In the invention, the X-ray powder diffraction spectrum was measured with use of RINT2000/PC manufactured by Rigaku International Corporation.

### Process for Preparing TAZB of the Invention

The crystalline penicillin of the invention can be prepared by adding TAZB in the form of oily substance or in the form of amorphous powder to at least one species selected from alcohols and ketones to crystallize the TAZB.

TAZB in the form of oily substance or in the form of amorphous powder can be simply prepared by known processes disclosed, e.g., in Japanese Examined Patent Publication No.121949/1995, Japanese Unexamined Patent Publication No.53462/1996 and so on.

In the present invention, TAZB in the form of oily substance or in the form of amorphous powder is used usually as dissolved in a suitable organic solvent. Preferred organic solvents are those which can dissolve TAZB and which are highly compatible with alcohol or ketone such as methylene chloride and like hydrocarbon halides, dimethylformamide and like amides, dioxane, tetrahydrofuran and like ethers, etc. These organic solvents can be used either alone or in combination.

The amount of the organic solvent to be used is not limited and is in the range which can dissolve TAZB and which does not adversely affect the operations to be later carried out. For example, the organic solvent is used in an amount of about 10 to about 30 liters, preferably about 15 to about 25 liters, per kilogram of TAZB.

In the present invention, it is possible to use, as the raw material, an organic solvent solution containing unpurified TAZB prepared by known processes disclosed in the foregoing publications, i.e. Japanese Examined Patent Publication No.121949/1995 and Japanese Unexamined Patent Publication No.53462/1996.

It is preferable in the present invention to concentrate an organic solvent solution containing TAZB in the form of oily substance or in the form of amorphous powder or an organic solvent solution containing unpurified TAZB to increase the operational efficiency in crystallization and the crystallization efficiency in the ensuing operation. The concentration is conducted before TAZB is mixed with alcohol and/or ketone. While the extent of concentration is not limited, the concentration is usually continued until the amount of the solution is reduced to about 1/2 to about 1/10 the amount before concentration.

In this invention, TAZB in the form of oily substance or in the form of amorphous powder can be used without being dissolved in an organic solvent.

The kind of alcohol which is used in crystallization is not limited and includes, for example, preferably methanol, ethanol, propanol, isopropanol, butanol and like alcohols having 1 to 4 carbon atoms, more preferably methanol, ethanol, isopropanol and like alcohols having 1 to 3 carbon atoms. Alcohol to be used may contain water. The content of water in the mixture of water and alcohol is about 10 (v/w)% or less, preferably about 5 (v/w)% or less.

The kind of ketone to be used in crystallization is not limited and includes, for example, preferably acetone, methyl ethyl ketone, methyl isobutyl ketone and the like which have same or different kinds of two alkyl groups of 1 to 4 carbon atoms substituted, more preferably acetone.

The amount of at least one species selected from alcohols and ketones which is used is not limited. Generally about 100 to about 10000 parts by weight of alcohol or the like is used per 100 parts by weight of the solution when using a solution of TAZB in an organic solvent from the viewpoint of efficient crystallization. When using TAZB in the form of oily substance or in the form of amorphous powder, alcohol or the like is used in an amount of about 5 to about 20 liters per kilogram of TAZB.

TAZB is crystallized by mixing TAZB in the form of oily substance and/or in the form of amorphous powder or an organic solvent solution thereof with at least one species selected from alcohols and ketones. The TAZB-crystallization efficiency can be increased by concentrating the resulting mixture and cooling the concentrate.

The temperature condition for crystallization are not limited and usually about 10°C or lower, preferably about 5°C or lower. The crystallized TAZB can be easily isolated from the reaction system and purified by known separation means such as filtration, concentration, drying under reduced pressure or the like. For example, the drying is carried out at a temperature of about 25 to about 40°C under reduced pressure of about 30 to about 0.1 kPa.

The crystalline penicillin of the invention thus obtained is white crystal having the above X-ray diffraction pattern.

The crystalline penicillin of the invention can be made into tazobactam useful as a beta-lactamase inhibitor, for example, by the process disclosed in Japanese Patent No.2648750 illustrated by the reaction scheme given below.

### Reaction Scheme

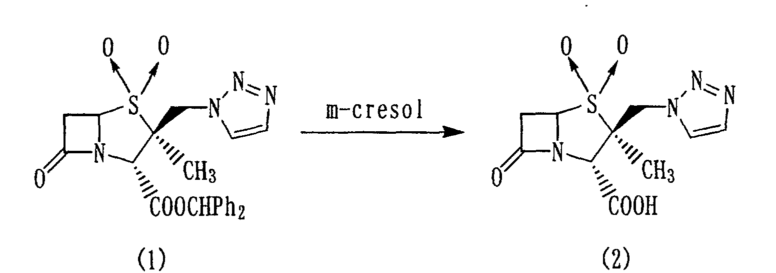

wherein Ph is as defined above.

### Best Mode for Carrying Out the Invention

The present invention will be described below in more detail with reference to the following examples. However, the scope of the invention is not limited by these examples.

### Example 1

A 1-liter eggplant-type flask was charged with 30 g of diphenylmethyl 2-methyl-2-triazolylmethylpenam-3-carboxylate 1,1-dioxide in the form of amorphous powder, and 580 ml of methylene chloride to give a solution. The methylene chloride was concentrated under reduced pressure.

The methylene chloride distilled off by concentration was recovered as a liquid by passing through a condenser in which a refrigerant (-5 to -20°C) was refluxed. When the amount of the recovered liquid reached about 420 ml, 400 ml of methanol was added. The concentration was continued until the amount of recovered organic solvent reached about 200 ml. Thereafter the crystal of diphenylmethyl 2-methyl-2-triazolylmethylpenam-3-carboxylate 1,1-dioxide was precipitated by 1-hour stirring while retaining the concentrate at a temperature at 5° C or lower.

The precipitate was collected by filtration under reduced pressure and was washed with methanol. The precipitate was dried under reduced pressure at about 40°C, giving 28.5 g of crystal of diphenylmethyl 2-methyl-2-triazolylmethylpenam-3-carboxylate 1,1-dioxide, which was found to have a purity of 100% as measured by HPLC.

The X-ray powder diffraction pattern of the crystal obtained by copper radiation of λ=1.5418 angstroms through a monochromator was measured, and was found to have high peak intensity at the following interplanar spacings.

| d (interplanar spacing) | (Relative intensity) |
|---|---|
| 10.9876 | 0.44 |
| 7.2251 | 1.00 |
| 5.4668 | 0.71 |
| 5.1681 | 0.77 |
| 4.9186 | 0.16 |
| 4.6768 | 0.17 |
| 4.3710 | 0.31 |
| 4.2915 | 0.35 |
| 3.9345 | 0.29 |
| 3.7386 | 0.31 |
| 3.2338 | 0.28 |

The ¹H-NMR spectrum data of the obtained crystal are as follows.
¹H-NMR (CDCl₃)δ ppm:
1.05 (s, 3H), 3.54 (m, 2H), 4.61 (m, 1H), 4.66 (s, 1H), 5.10 (dd, J=12, 15Hz, 2H), 7.00 (s, 1H), 7.36 (s, 10H), 7.72 (s, 1H), 7.74 (s, 1H)

### Example 2

A 1-liter eggplant-type flask was charged with 600 ml of a methylene chloride solution containing about 30 g of diphenylmethyl 2-methyl-2-triazolylmethylpenam-3-carboxylate 1,1-dioxide(TAZB). The methylene chloride was concentrated under reduced pressure. The methylene chloride distilled off by concentration was recovered as a liquid by passing through a condenser in which a refrigerant (-5 to -20°C) was refluxed. When the amount of the recovered liquid reached about 420 ml, 400 ml of methanol was added. The concentration was continued until the amount of recovered organic solvent reached about 200 ml. Thereafter the crystal of diphenylmethyl 2-methyl-2-triazolylmethylpenam-3-carboxylate 1,1-dioxide was precipitated by 1-hour stirring while retaining the concentrate at a temperature at 5°C or lower.

The precipitate was collected by filtration under reduced pressure and was washed with methanol. The precipitate was dried under reduced pressure at about 40°C, giving 29.0 g of crystal of diphenylmethyl 2-methyl-2-triazolylmethylpenam-3-carboxylate 1,1-dioxide, which was found to have a purity of 100% as measured by HPLC.

The X-ray powder diffraction pattern of the crystal obtained by copper radiation of λ=1.5418 angstroms through a monochromator was measured, and was found to have high peak intensity at the following interplanar spacings.

| d (interplanar spacing) | (Relative intensity) |
|---|---|
| 10.9604 | 0.43 |
| 7.2251 | 1.00 |
| 5.4668 | 0.55 |
| 5.1691 | 0.62 |
| 4.9132 | 0.18 |
| 4.6768 | 0.16 |
| 4.3710 | 0.28 |
| 4.2956 | 0.27 |
| 3.9345 | 0.25 |
| 3.7355 | 0.28 |
| 3.2338 | 0.25 |

### Reference Example 1 (Preparation of tazobactam crystal)

Ten g of crystals of diphenylmethyl 2-methyl-2-triazolylmethylpenam-3-carboxylate 1,1-dioxide obtained in Example 1 was added to 80 ml of m-cresol heated to 50 to 55°C. A reaction was performed for 2 hours while maintaining the temperature.

After completion of the reaction, 240 ml of methyl isobutyl ketone was added and the mixture was cooled to 0 to 5°C. Added thereto were 23 ml of water and 2.3 g of sodium hydrogencarbonate for extraction. The organic layer was separated. To the organic layer were added 12 ml of water and 0.7 g of sodium hydrogencarbonate for further extraction. The aqueous layers separated were washed together with 18 ml of methyl isobutyl ketone and cooled to O to 5°C. 6N hydrochloric acid was added to adjust the pH to 1 or less. The precipitated tazobactam was collected by filtration, washed with a small amount of cold water and dried, whereby white crystal of tazobactam was obtained (yield 95%).

### Comparative Example 1

A 1-liter 4-necked flask was charged with about 260 ml of a methylene chloride solution containing 29 g of diphenylmethyl 2-methyl-2-triazolylmethylpenam-3-carboxylate 1,1-dioxide(TMPB). To the solution were added 120 ml of a 90% aqueous solution of acetic acid and 20 g of potassium permanganate. The mixture was stirred at about 42°C for 3 hours. Added thereto were 340 ml of methylene chloride and 180 ml of water. The mixture was cooled to 5°C and 24 ml of 35% hydrogen peroxide was added dropwise in a manner to take care of bubbling. The aqueous layer was discarded away and the organic layer was washed successively with a 2% aqueous solution of sodium bisulfite and with water. The organic layer was dried and the methylene chloride was concentrated under reduced pressure, whereby 37.4 g (TAZB content 79%) of an oily substance was obtained.

### Comparative Example 2

A 1-liter 4-necked flask was charged with about 260 ml of a methylene chloride solution containing 29 g of diphenylmethyl 2-methyl-2-triazolylmethylpenam-3-carboxylate(TMPB). To the solution were added 120 ml of a 90% aqueous solution of acetic acid and 20 g of potassium permanganate. The mixture was stirred at about 42°C for 3 hours. Added thereto were 340 ml of methylene chloride and 180 ml of water. The mixture was cooled to 5°C and 24 ml of 35% hydrogen peroxide was added dropwise in a manner to take care of bubbling. The aqueous layer was discarded away and the organic layer was washed successively with a 2% aqueous solution of sodium bisulfite and with water. The methylene chloride was concentrated under reduced pressure. When about 500 ml of methylene chloride was distilled off, a solvent mixture of 100 ml of ethyl acetate and 100 ml of n-hexane was added. The precipitate was filtered under reduced pressure and washed with a solvent mixture of 20 ml of ethyl acetate and 20 ml of n-hexane. The precipitate was dried under reduced pressure at about 40°C, giving 31.6 g (TAZB content 93%) of amorphous powder of diphenylmethyl 2-methyl-2-triazolylmethylpenam-3-carboxylate 1,1-dioxide.

### Reference Example 2

A test tube was charged with 10 g of each of the TAZB crystal of Example 1 (purity 100%), the TAZB crystal of Example 2 (purity 100%), the TAZB oily substance of Comparative Example 1 (purity 79%), and the TAZB amorphous powder of Comparative Example 2 (purity 93%). The test tubes were sealed and stored at room temperature (5 to 35°C) for 1 year after which their purity was determined by HPLC.

The results are as follows: the purity of TAZB crystal of Example 1 was 99%, the purity of TAZB crystal of Example 2 was 99%, the purity of TAZB oily substance of Comparative Example 1 was 52%, and the purity of TAZB amorphous powder of Comparative Example 2 was 81%.

The crystalline TAZB of the invention had a purity-lowering ratio of 1% or less, when stored at room temperature for 1 year.

## Claims

1. A crystal of diphenylmethyl 2-methyl-2-triazolylmethylpenam-3-carboxylate 1,1-dioxide having peaks at the following interplanar spacings in the X-ray powder diffraction pattern obtained by copper radiation of λ=1.5418 angstroms through a monochromator.
| d (interplanar spacing) |
|---|
| 10.412-11.508 |
| 6.864-7.586 |
| 5.193-5.740 |
| 4.911-5.428 |
| 4.668-5.159 |
| 4.443-4.911 |
| 4.152-4.590 |
| 4.081-4.510 |
| 3.738-4.131 |
| 3.549-3.922 |
| 3.072-3.395 |

2. The crystalline diphenylmethyl 2-methyl-2-triazolylmethylpenam-3-carboxylate 1,1-dioxide according to claim 1, wherein the X-ray powder diffraction pattern obtained by copper radiation of λ=1.5418 angstroms through a monochromator is as follows:
| d (interplanar spacing) | I/Io(relative intensity) |
|---|---|
| 10.412-11.508 | 0.42-0.45 |
| 6.864-7.586 | 1.00 |
| 5.193-5.740 | 0.39-0.87 |
| 4.911-5.428 | 0.47-0.92 |
| 4.668-5.159 | 0.14-0.20 |
| 4.443-4.911 | 0.15-0.18 |
| 4.152-4.590 | 0.25-0.34 |
| 4.081-4.510 | 0.19-0.43 |
| 3.738-4.131 | 0.21-0.33 |
| 3.549-3.922 | 0.25-0.34 |
| 3.72-3.395 | 0.22-0.31 |

3. The crystalline diphenylmethyl 2-methyl-2-triazolylmethylpenam-3-carboxylate 1,1-dioxide according to claim 1 or 2 which is obtainable by adding diphenylmethyl 2-methyl-2-triazolylmethylpenam-3-carboxylate 1,1-dioxide represented by the formula (1) 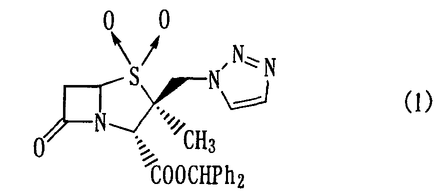 wherein Ph is a phenyl group, in the form of oily substance or in the form of amorphous powder or an organic solvent solution thereof to at least one species selected from alcohols and ketones to crystallize the diphenylmethyl 2-methyl-2-triazolylmethylpenam-3-carboxylate 1,1-dioxide, the crystalline compound being stable substantially without decomposition or degradation of properties even when left to stand at a temperature of 5 to 35°C for a period of 1 year.

4. A process for preparing the crystalline diphenylmethyl 2-methyl-2-triazolylmethylpenam-3-carboxylate 1,1-dioxide according to claim 1, the process comprising the step of adding diphenylmethyl 2-methyl-2-triazolylmethylpenam-3-carboxylate 1,1-dioxide represented by the formula (1) 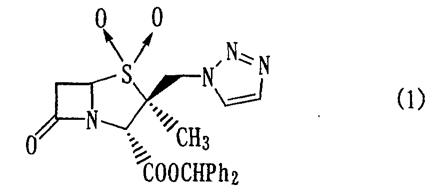 wherein Ph is a phenyl group, in the form of oily substance or in the form of amorphous powder or an organic solvent solution thereof to at least one species selected from alcohols and ketones to crystallize the diphenylmethyl 2-methyl-2-triazolylmethylpenam-3-carboxylate 1,1-dioxide.
